# EUROPEAN PATENT APPLICATION

(11) **EP 0 634 154 A1**
(43) Date of publication of application: **18.01.1995**
(21) Application number: 94830357.3
(22) Date of filing: 14.07.1994
(51) Int. Cl.: A61F 2/36

(54) **Improvement in the locking among the components of a multicomponent prosthesis for use in traumatology, orthopedics and veterinary medicine**

(30) Priority: 16.07.1993 IT BS930080
(71) Applicant: MEC HINT S.r.l., I-24060 Castelli Calepio (Bergamo) (IT)
(72) Inventor: Monfardini, Ennio Alessio, I-25036 Palazzolo S/Oglio (Brescia) (IT); Grassi, Angelo, I-25036 Palazzolo S/Oglio (Brescia) (IT)
(74) Representative: Manzoni, Alessandro

(57) **Abstract**

The present invention pertains to a multicomponent prosthesis for use in traumatology, orthopedics and veterinary medicine for joint prosthesis operations, which consists of at least a first component (11, 111) and a second component (112) [sic, (12, 112) - Tr.Ed.] which are connected by a first conical coupling (15, 115; 21, 121) and a second conical coupling (23, 26; 123, 126), which is coaxial and is tapered in the opposite direction to that of the first, conical coupling The second conical coupling is formed by a conical seat (23, 123) made in the second component (12, 112) of the prosthesis, by a female friction cone (13, 113) inserted into the conical seat (11, 111) [sic, (23, 123) - Tr.Ed.] and by a locking counterscrew (14) which is screwed onto the first component and interacts axially with the female friction cone (13) in order to immobilize it in the conical seat.

## Description

The present invention pertains, in particular, to hip prostheses, but it can also be applied in other joint prosthesis operations, such as those for the shoulder, the knee, etc. More specifically, the present invention pertains to the joining among the components of the shaft of a prosthesis to be mounted in the medullary pin, if necessary. which is suitably adapted and bears the joint head, and when the said shaft necessarily consists of two or more complementary parts.

The joint prosthesis operation as concerns the hip is usually carried out by resectioning the femoral head, adapting the proximal medullary pin of the femur, and then by fixing a prosthesis using, if considered valid, a special cement; the completed unit with the mounting of a spherical head is intended to replace the natural femoral head.

Various types of prostheses, having various geometric shapes and being made with various types of alloys and/or metals, are available in the fields of orthopedics, traumatology and veterinary medicine. It is almost always felt to be necessary to have available a prosthesis that is suitable for each femur, and when this is indispensable, one resorts to making personalized prostheses by designing them with the aid of computerized axial tomography (CAT), but with a resulting, very often unacceptable, bearing on the costs. Therefore, in order to avoid modifications to the normal single-piece prostheses, which can really only succeed thanks to the high professional skill of the surgeon one would have to resort to a two-component or three-component prosthesis.

In other words and for example, if the distal part is separated from the proximal part of a prosthesis shaft and/or the upper headholder part of the said proximal part, the different components can be selected as a function of the geometry of the skeleton on which the operation is being performed, reducing the bone removal as much as possible by means of adjustment and avoiding irreparable damage.

Therefore, it could be possible to achieve the distribution of loads on the femur and thus the different stresses due to the forces that act on the prosthesis in various directions, under as natural conditions as possible, and with easily imaginable advantages for the individual, certainly limiting costs and tremendously facilitating the operation, and shortening the postoperative course and the rehabilitation times. For this reason, proposals have already been made for multicomponent prostheses, which, however, have shown great shortcomings in the connecting and locking means of the various parts, especially due to the possibility of demobilization of the components, which can be confirmed over time as a result of the fatigue stresses to which they are subjected and which are not easily foreseeable.

The purpose of the present invention is to eliminate these shortcomings by means of a new, original system of joining and locking the components of a multicomponent prosthesis among one another, a system which makes it possible to support all the stresses that may occur without loosenings and therefore to maintain a correct and reliable locking of the connected components over time.

Another purpose of the present invention is to provide a system of joining and locking among the components of a multicomponent prosthesis having simple, practical handling and thus easy access and use by the surgeon.

The present invention proposes to achieve these objectives with a prosthesis which consists of at least a first component and a second component which are connected at the adjoining ends by a first conical coupling which is tapered in one direction and is formed by a conical end at one end of the first component inserted in a conical housing at the adjoining end of the second component, and which is characterized by the fact that it has a second conical coupling which is coaxial and is tapered in the opposite direction to that of the first conical coupling and is formed by a conical seat made in the second component of the prosthesis, and by a female friction cone which is applied and is axially adjustable on the first component, and a locking counterscrew which is screwed onto the first component and interacts axially with the said female friction cone in order to immobilize it in the conical seat of the second component.

The attached drawings illustrate an exemplary embodiment of a hip prosthesis having two components. The detailed description below is given with reference to such an example, taking into account, however, that the same locking system can be used with more than two components and as stated above, for prostheses intended for operations on the shoulder, the knee, etc. as well. In the said drawings,
Figure 1 shows an exploded view of a prosthesis with two components according to the present invention;
Figure 2 shows the assembly of the two components in longitudinal and partial section;
Figure 3 shows the exploded view of a prosthesis with three components; and
Figure 4 shows the assembly of the prosthesis of Figure 3 in longitudinal section.

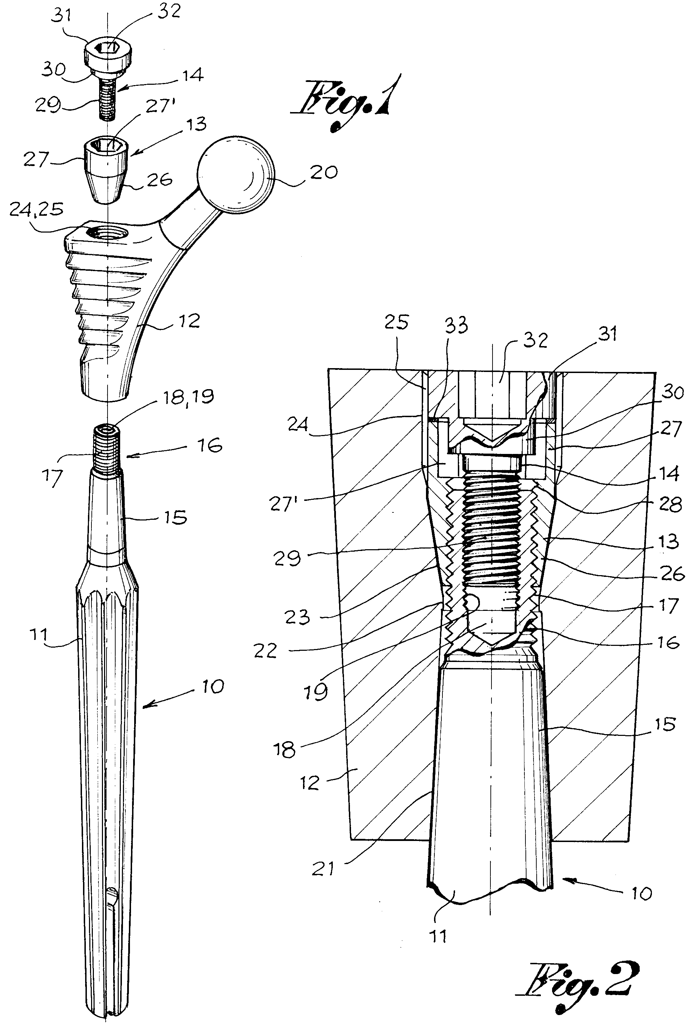

In the embodiment shown in Figures 1 and 2, the prosthesis has a shaft 10 consisting of a first distal component 11 and a second proximal component 12, which are intended to be coupled in a complementary manner and to be locked by means of a female friction cone 13 and a counterscrew 14. The said distal component 11, which can have the most suitable shape for being more readily adapted to the proximal pin of the femur, ends in its part turned towards the said proximal component 12 with a "Morse"-type conical end 15, and a cylindrical shank 16 is accommodated at the top of the said conical end 15. The said shank 16 has an external thread 17 and an axial hole 18 with an internal thread 19. The said external thread 17 and the said internal thread 19 preferably have pitches that are different from one another.

The said proximal component 12 can have the most suitable and desired shape and usually bears a spherical head 20 which replaces the natural femoral head. In the embodiment shown in Figures 1 and 2 as well, such a component 12 is provided, in its part turned towards the said distal component 11, with a conical housing 21, which is intended to accommodate the said conical end 15 of the said distal component 11 with suitable tolerance. Also in the said component 12, starting from the most tapered part of the said conical housing 21 up to the upper part, or the most proximal part, of the said component, are provided in the following order: an intermediate cylindrical hole section 22, a conical seat 23 which is tapered in the opposite direction to that of the said conical housing 21, and an end cylindrical hole section 24 with or without an internal thread 25.

The said locking female friction cone 13 has a lower conical part 26, an upper cylindrical part 27 and an axial threaded hole 28 through which the said female friction cone 13 is screwed onto the said external thread 17 of the said shank 16 of the said distal component 11. Therefore, the said conical part 26 of the said female friction cone 13 is intended to be coupled with the said conical seat 23 in the said proximal component 12 in opposition to the insertion of the said conical end 15 of the said distal component 11 into the conical housing of the said proximal component. A hexagonal slot 27' is provided on the head of the said cylindrical part 27 for an operating wrench or screw connection by means of the said female friction cone 13 until the components are axially tightened.

The said counterscrew 14 has a threaded shank 29 with a thread corresponding to that (19) of the said axial hole 15 in the said shank 16 of the said distal component, and a head with two cylindrical sections 30, 31, which is adjacent to the said shank 29, which has a smaller diameter than the said head. A hexagonal slot 32 is also provided in the head of the said counterscrew 14 for a corresponding operating wrench.

Once the said female friction cone 13 is screwed onto the said external thread 17 of the said shank 16 of the said distal component 11, the said counterscrew 14 is screwed into the threaded axial hole of the said shank 16, as shown in Figure 2. Thus, the said smaller cylindrical section 30 of the head of the said counterscrew 13 [sic, 14 - Tr.Ed.] is sunk into the hexagonal slot 27' of the said female friction cone, and the base of the said larger section 31 is supported and engages on the top of the said female friction cone, preventing any possibility of its unscrewing, and thus loosening of the locking.

Therefore, the assembly of the said components 11, 12 of the prosthesis is ensured by the two coaxial conical couplings (15, 21; 23, 26), with opposite smaller bases, and by the said counterscrew 14 which stabilizes the locking. All coupling clearances are also eliminated with a suitable selection of the tapering of these couplings. The said counterscrew 14 acts on the said female friction cone 13 like a lock nut on a screw nut, by stressing the turns of the male screws always in the same direction and never releasing them, but rather always keeping them under force. In addition, the adoption of threads with a different pitch on the outside and on the inside of the said shank 16 of the said distal component and correspondingly for the said female friction cone 13 and the said counterscrew 14, intended more for the said female friction cone 13 than for the said counterscrew 14, ensures that, if microvibrations were to be created in the system, or if the female friction cone were, in any manner, stressed to be unscrewed, there would be a double locking by the screw deriving from the function of the counterscrew itself and from the different pitch of the thread.

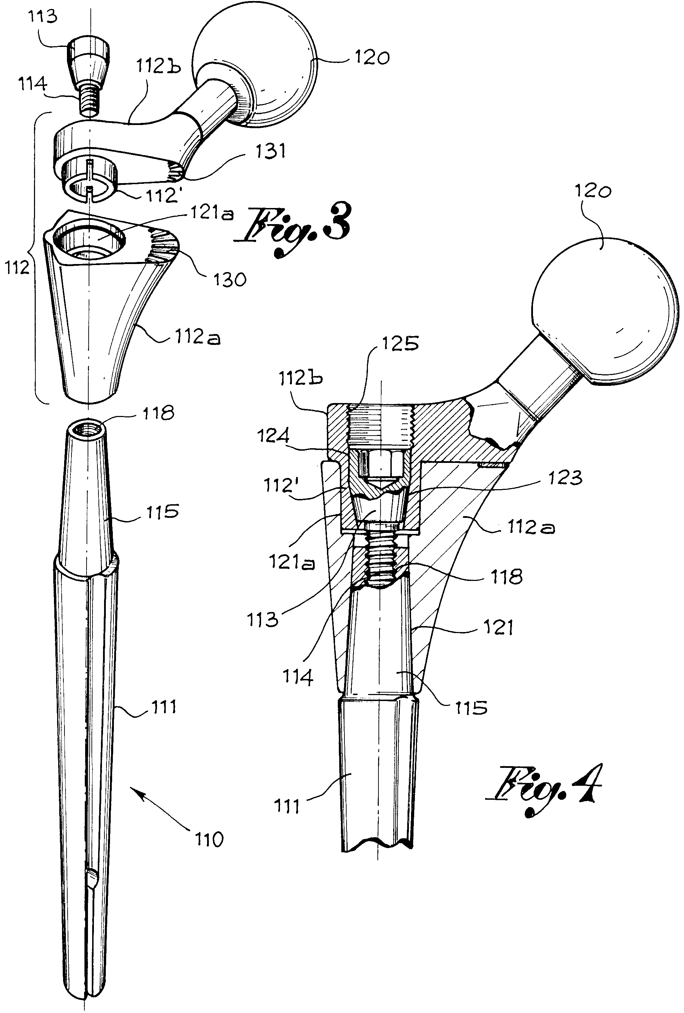

In the embodiment shown in Figures 3 and 4, the distal component 111, which lacks the threaded shank at the end, has an axial hole 118 on the head of the conical end 115, and the proximal component 112 consists of two complementary sections 112a, 112b. The said first section 112a has a conical housing 121 intended to accommodate the said conical end 115 of the said distal component 111, followed by a cylindrical seat 121a. The said other section 112b bears a common spherical head 120 and is intended to be connected with the said first section 112a. This second section 112b has an expandable base neck 112', which is split radially and is intended to engage in the cylindrical seat of the said section 112a, axially to the said neck 112', a conical hole 123 which is tapered in the opposite direction to that of the said housing 121 in the said first section 112a, and a cylindrical hole 124 with an internal thread 125, at least on one part.

This three-component prosthesis 111, 112a, 112b can be assembled with the same female friction cone 13 and the said counterscrew 14 described above with reference to Figures 1 and 2. As an alternative for the assembly of the prosthesis, an integrated element consisting of a locking 113 and of a counterscrew 114, which form an integral, single piece, can be used. This integrated element 113, 114 shall be preferably made of a material with the capacity for storing heat, and since it is screwed onto the distal component itself, it determines an expansion of the said expandable neck 112' which ensures the connec- tion of the various parts. Finally, in the case of the proximal component in the two sections 112a, 112b, the contact surfaces between the said two sections 112a, 112b of the said proximal component 112 consist of toothings 130, 131, respectively, which make it possible to vary the orientation of one section with respect to the other section and which, being inserted when the said components 111 and 112 are locked by means of the said separated or integrated female friction cone (13, 113) and counterscrew (14, 114), ensure a fixed locking of the said two portions 112, 112b [sic, 112a, 112b - Tr.Ed.] despite the multidirectional forces that are released on the prosthesis during its use.

However, with the system described above, detail modifications can be introduced without thereby going beyond the scope of the present invention. Thus, for example: the said conical end part 15, 115 of the said distal component 11, 111 may be partly grooved or toothed and may have a grooved cylindrical part, a spring washer 33 may be arranged between the top of the said female friction cone 13 and the base of the larger section 31 of the head of the said counterscrew 14, and additional components against unscrewing, which are radial or tangential may be connected to the said counterscrew and/or to the said female friction cone.

## Claims

1. Multicomponent prosthesis for use in traumatology, orthopedics and veterinary medicine for joint prosthesis operations, consisting of at least a first component (11, 111) and a second component (112), which are connected at the adjoining ends by a first conical coupling (15, 115; 21, 121) which is tapered in one direction and is formed by a conical end (15, 115) at one end of the said first component (11, 111) which is inserted into a conical housing (21, 121) at the adjoining end of the said second component, characterized by a second conical coupling (23, 26; 123, 126), which is coaxial and is tapered in the opposite direction to that of the said first conical coupling, and which is formed by a conical seat (23, 123) made on the said second component (12, 112) of the prosthesis, by a female friction cone (13, 113), which is applied and is axially adjustable in the said second component, and by a locking counterscrew (14) which is screwed onto the said first component and interacts with the said female friction cone (13) in order to immobilize it in the conical seat of the said second component.

2. Multicomponent prosthesis in accordance with claim 1, in which the first component (11) and the second component (12) are the distal part and the proximal part, respectively, of a hip prosthesis or for another operation, with a spherical head being able to be fixed to the said second component.

3. Multicomponent prosthesis in accordance with claim 1, in which a shank (16) is provided at the smaller end of the conical end (15) of the first component (11), which [shank] has an external thread (17) and an axial hole (18) with an internal thread (19), in which, in the second component (12), the conical seat (23) for the female friction cone (13) is opposite the conical housing (21) for the said conical end (15) of the first component and ends from the opposite part of the said conical housing (21) with a cylindrical hole section (24), in which the female friction cone (13) is screwed onto the said external thread (17) of the said shank (16) in order to engage in the said conical seat (23) in opposition to the action of the said conical end (15) in the said conical housing (21), and in which the said counter-screw (14) is screwed into the threaded axial hole (18, 19) of the said shank (16) and bears a head (30, 31) which is supported on the said female friction cone (13).

4. Multicomponent prosthesis in accordance with claim 3, in which the external thread (17) and the internal thread (19) of the said shank (16) of the first component (11) have the same pitch.

5. Multicomponent prosthesis in accordance with claim 3, in which the external thread (17) and the internal thread (19) of the said shank (16) of the first component (11) have a different pitch.

6. Multicomponent prosthesis in accordance with one of the above claims, in which a spring washer (33) is arranged between the female friction cone (13) and the counterscrew (14).

7. Multicomponent prosthesis in accordance with claims 1 to 5, in which the said female friction cone (113) and the said counterscrew (114) are integrated in order to form a single piece.

8. Multicomponent prosthesis in accordance with any of the above claims, in which the second component (112) consists of two sections (112a, 112b) which are coupled in a complementary manner by means of at least partially toothed contact surfaces (130, 131), a first section (112a) having a conical housing (121) for accommodating the conical end (115) of the first component (11), while the second section (112b) bears a spherical head (120) and is fixed on the first section by means of screwing the female friction cone (13, 113)-counterscrew (14, 114) unit onto the first component.

9. Multicomponent prosthesis in accordance with claim 8, in which the first section (112a) has, axially to the conical housing (121), a cylindrical seat (121a), and in which the second section (112b) has an expandable base neck (112') intended to be inserted and to be locked in the said cylindrical seat (121a) as a result of screwing the female friction cone (13, 113)-counterscrew (14, 114) unit onto the first component.

10. Multicomponent prosthesis in accordance with one of the above claims, in which part of the conical end (15) of the first component is grooved or toothed on the periphery or is integrated with a cylindrical section that is grooved or toothed on the periphery.

11. Multicomponent prosthesis in accordance with the above claims, comprising, in addition, at least a means for radially or tagentially locking the said female friction cone and/or the said counterscrew against unscrewing.
